# EUROPEAN PATENT SPECIFICATION

(11) **EP 0 546 393 B1**
(45) Date of publication and mention of the grant of the patent: **20.03.1996**
(21) Application number: 92120237.0
(22) Date of filing: 26.11.1992
(51) Int. Cl.: B01F 3/08, C07K 1/04

(54) **Peptide synthesizer**
Vorrichtung zur Herstellung von Peptiden
Synthétiseur de peptides

(30) Priority: 26.11.1991 JP 337531/91
(43) Date of publication of application: 16.06.1993
(73) Proprietor: SHIMADZU CORPORATION, Nakagyo-ku Kyoto-shi Kyoto 604 (JP)
(72) Inventor: Nokihara, Kiyoshi, Kyoto-shi, Kyoto (JP); Hazama, Makoto, Kyoto-shi, Kyoto (JP); Yamamoto, Rintaro, Kyoto-shi, Kyoto (JP)
(74) Representative: WILHELMS, KILIAN & PARTNER Patentanwälte

(56) References cited:
- DE-C- 890 728
- FR-A- 2 156 204
- Section Ch, Week 8903, 1 March 1989 Derwent Publications Ltd., London, GB; Class J01, AN 89-021780

## Description

### FIELD OF THE INVENTION

The present invention relates to an apparatus for dissolving an amino acid derivative in a peptide synthesizer equipped with an amino acid container.

### BACKGROUND OF THE INVENTION

In ordinary peptide synthesizers, a solution of an amino acid derivative activated for coupling, prepared by dissolving a powdery acyl component in an amino acid container, is subjected to the coupling reaction. When using an automatic peptide synthesizer, this amino acid derivative is automatically dissolved in the presence of a solvent such as dimethylformamide. However, because the solvent is usually dropwise added, the amino acid derivative in a solid state becomes lumpy in the amino acid container, and a part of it often remains insoluble like gum status in the container. Therefore, in the peptide synthesis, even when aspiration of the amino acid derivative solution is attempted, it is not possible to aspirate the specified amounts of amino acid into the nozzle, or the nozzle tip is obstructed by lumps, or PyBOP® (benzotriazole-1-yloxy-tris-pyrrolidino-phosphonium hexafluorophosphite) and HOBt (N-hydroxybenzotriazole), which are more soluble than the amino acid derivative, NMM (N-methylmorpholine), which is liquid at normal temperature, and other substances enter in the reaction vessel in excess. As a result, the balance of stoichiometry of the reaction becomes poor, so that side reactions may undesirably take place. These can cause hardware troubles, the production of deletion peptides due to incomplete reaction, and various side reactions, which result in problems such as lowered yield and purity of the desired final product and difficulty in purification. Although these problems can be solved by constantly confirming the use of the completely dissolved amino acid derivative, it wastes time. Therefore, a more satisfactory means for dissolution has been demanded.

Means for dissolving an amino acid derivative include heating, shaking and nitrogen bubbling. The present inventors have made investigations in search for an efficient and simple means which can be housed in the limited space of the peptide synthesizer, and found that good mixing by stirring can be achieved in a combination of a nitrogen bubbling and particular actions of a needle. The present inventors have made further investigations based on this finding, and thus developed the present invention.

### SUMMARY OF THE INVENTION

Accordingly, the present invention is directed to an apparatus for dissolving an amino acid derivative comprising a container for dissolving an amino acid derivative in a short time, a bubbling nozzle for discharging gas into the inside of the container and means for moving the nozzle in the container.

### BRIEF DESCRIPTION OF THE DRAWINGS

Figure 1 is a schematic diagram of an amino acid container with a bubbling nozzle inserted therein according to the present invention; and
Figure 2 shows a reverse-phase HPLC profile of crude porcine neuromedin U, a twenty-five amino acid peptide, after cleavage without purification.

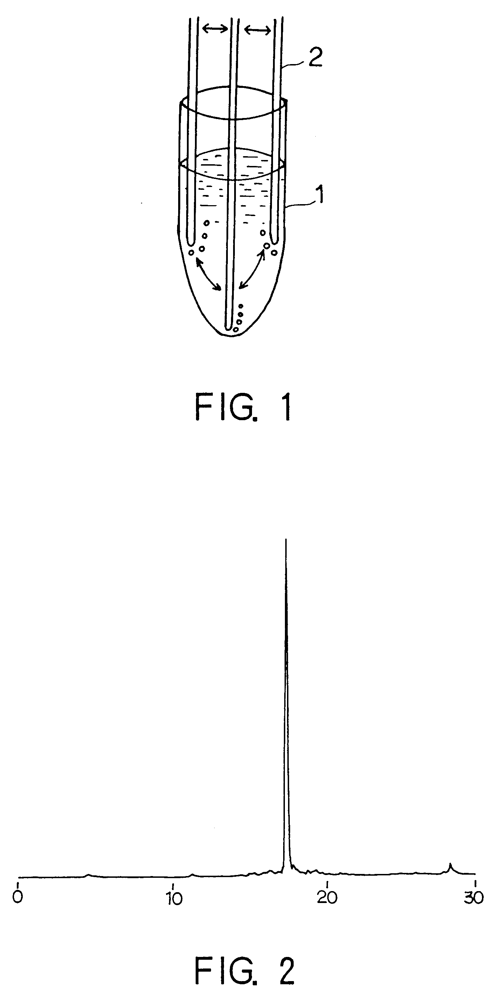

The reference numerals in Figure 1 denote the following elements:
Element 1 is an amino acid container, and element 2 is a needle with a bubbling nozzle.

### DETAILED DESCRIPTION OF THE INVENTION

In the present invention, the bubbling nozzle is inserted in the amino acid container, and the bubbling takes place while laterally and vertically moving the nozzle tip along the inner wall of the container to dissolve the amino acid derivative in the same manner as using, for example, spatel by manual operation. The bubbling gas discharged from the nozzle is thus diffused uniformly in the container. Even when the amino acid derivative is difficult to dissolve and becomes gummy, the dissolution is facilitated to prevent the amino acid derivative from adhering to the needle and/or the inner wall of the container, thereby the complete dissolution can be achieved.

The peptide synthesizer of the present invention is detailed below, referring to the drawings. Figure 1 is a schematic diagram of an amino acid container with a bubbling nozzle inserted therein according to the present invention.

An amino acid derivative and a reagent for activation such as PyBOP® together with a reactant such as HOBt and NMM are placed in an amino acid container 1 as illustrated in Figure 1. A needle with a bubbling nozzle 2 is moved along the inner wall of the amino acid container 1 while discharging gas, thereby all of the amino acid derivatives can be completely dissolved and preactivated.

Specifically, in the present invention, nitrogen gas is usually used as the bubbling gas, whose flow rate can be selected appropriately, for instance, about 12 to 15 ml/minutes. The bubbling nozzle is moved together with the needle. The apparatus is capable of controlling the nitrogen gas flow rate from the bubbling nozzle by means such as an electromagnetic valve. The needle is connected to a movable arm, which swings not only laterally but also vertically by moving the nozzle tip along the inner wall of the container to dissolve the amino acid derivative in the same manner as using, for example, spatel by manual operation, and the movable arm is driven by a motor via a pulley or a timing belt. The needle moves by such means for moving the bubbling nozzle in the amino acid container. For instance, the needle is reciprocated vertically twice, while laterally once in the amino acid container, thereby moving the bubbling nozzle tip along the inner wall of the amino acid container. These movements of the needle are controlled by a motor. The moving speed of the bubbling nozzle is appropriately selected. The nozzle swings not only laterally but also vertically in the container, so as to rub off the amino acid derivative in gum status adhered to the inner wall of the container and/or the needle of the bubbling nozzle therein. The arrow illustrated in Figure 1 indicates the direction of the bubbling nozzle movement.

The apparatus for dissolving an amino acid derivative according to the present invention is associated with the reaction vessel arranged in a peptide synthesizer, so that the dissolved and activated amino acid derivative can be subjected to the reaction vessel for coupling.

The apparatus for dissolving an amino acid derivative is applicable to any of the conventionally known automated peptide synthesizers, in addition to any synthesizers for other than peptides, and thus not being limitative to particular ones. For instance, the apparatus is applicable to synthesizers for dissolving powdery reagents.

The apparatus of the present invention can thus prevent the amino acid derivative from adhering to the inner wall of the amino acid container and the needle by the combined action of the bubbling and the bubbling nozzle's movement to facilitate its dissolution and achieve the complete dissolution. Also, by using an amino acid derivative in the form of a uniform solution, a high-efficiency peptide synthesis can be accomplished rapidly and with excellent yield and purity.

### EXAMPLES

The present invention is hereinafter described in more detail by means of the following examples:

### Synthesis of porcine neuromedin U consisting of 25 amino acid residues

Each amino acid derivative and a PyBOP® reagent for activation were placed in an amino acid container 1 as illustrated in Figure 1, and a dimethylformamide (DMF) solution of HOBt and an NMM solution which were in separate bottles were added into the amino acid container. By means of an arm connected to a needle with a bubbling nozzle 2, the nozzle was moved along the inner wall of the amino acid container 1 while discharging gas, thereby all of the amino acid derivatives could be completely dissolved within a period of maximum 3 minutes. Also, the C-terminus of the amino acids was activated by the PyBOP®, HOBt and NMM for the coupling reaction at the next reaction chamber. The arm was driven by a motor. In the present example, the bubbling nozzle was laterally reciprocated once in two seconds. The amino acids were sequentially incorporated by active ester prepared in this apparatus to synthesize porcine neuromedin U consisting of 25 amino acid residues.

The protected peptide resin thus obtained was cleaved with a mixture of TFA containing appropriate scavengers (N-cocktail) at room temperature for 6 hours to provide the desired free peptide solution, which was filtered.

| Composition of N-Cocktail | |
|---|---|
| TFA (Trifluoroacetic acid) | 82.5 volume% |
| Ethyl methyl sulfide | 3 volume% |
| Water | 5 volume% |
| Thioanisole | 5 volume% |
| Ethanediol | 2.5 volume% |
| Thiophenol | 2 volume% |

Ether was added to the above filtrate (peptide in the cleavage cocktail as the reaction mixture) to allow precipitation. The resulting precipitate was collected using a centrifuge, dissolved in 30% acetic acid, 4 fold diluted with water and lyophilized to yield the desired peptide. The synthetic peptide thus obtained was identified by a reversed-phase HPLC as an almost single sharp peak as shown in Figure 2. The analytical conditions for reversed-phase HPLC used are as follows:
- Column:: SynProPep (Trademark) RPC18 (4.6 × 250 mm)
- Eluent:: 0.01 N hydrochloric acid/acetonitrile = 85/15 to 55/45 (30 minutes)
- Flow rate:: 1.0 ml/minute, absorbance: 210 nm
The yield was quantitative, and the purity was about 96%, calculated by UV absorption. This synthetic peptide had 25 amino acid residues and was identified as the desired porcine neuromedin U by the sequence analysis, the amino acid analysis and FAB mass spectrometry.

## Claims

1. An apparatus for dissolving an amino acid derivative in a peptide synthesizer comprising:
a container for dissolving an amino acid derivative;
a bubbling nozzle for discharging gas into the inside of the container; and
means for moving the nozzle in such a manner that said nozzle swings laterally and vertically in the container, so as to rub off said amino acid derivatives adhered to the inner wall of said container.

2. The apparatus according to claim 1, wherein said means is a movable arm, which swings laterally and vertically, said movable arm being connected to the needle and driven by a motor via a pulley or a timing belt.

## Patentansprüche

1. Vorrichtung zum Lösen eines Aminosäurederivats in einer Peptidsynthesevorrichtung mit:
einem Behälter zum Lösen eines Aminosäurederivats;
einer Einblasdüse zur Gaseinleitung in den Behälter; und
Mittel zum Bewegen der Düse derart, daß sie vertikal und seitlich im Behälter schwingen kann, um die Aminosäurederivate, die an der Innenwand des Behälters anhaften, abzureiben.

2. Vorrichtung nach Anspruch 1, wobei die Mittel ein beweglicher Arm sind, der seitwärts und vertikal schwingt, wobei der bewegliche Am mit der Düse verbunden und durch einen Motor mit einer Riemenscheibe oder einem Steuerriemen angetrieben wird.

## Revendications

1. Appareil pour la dissolution d'un dérivé d'aminoacide dans un synthétiseur de peptides, comprenant :
• un récipient pour la dissolution d'un dérivé d'aminoacide ;
• une buse de barbotage pour la décharge de gaz a' l'intérieur du récipient ; et
• un moyen pour mouvoir la buse de telle façon que ladite buse oscille latéralement et verticalement dans le récipient, de manière à enlever lesdits dérivés d'aminoacides adhérant à la paroi interne dudit récipient.

2. Appareil selon la revendication 1, dans lequel ledit moyen est un bras mobile oscillant latéralement et verticalement, ledit bras mobile étant relié à l'aiguille et commandé par un moteur par l'intermédiaire d'une poulie ou d'une courroie crantée.
